# EUROPÄISCHE PATENTSCHRIFT

(11) **EP 0 611 577 B1**
(45) Veröffentlichungstag und Bekanntmachung des Hinweises auf die Patenterteilung: **04.03.1998**
(21) Anmeldenummer: 93118280.2
(22) Anmeldetag: 11.11.1993
(51) Int. Cl.: A61M 1/00, A61M 15/00

(54) **Pulverinhalator**
Powder inhalator
Inhalateur de poudres

(30) Priorität: 24.11.1992 DE 4239402
(43) Veröffentlichungstag der Anmeldung: 24.08.1994
(73) Patentinhaber: BAYER AG, 51368 Leverkusen (DE)
(72) Erfinder: Herold, Heiko, Dipl.-Ing., D-41470 Neuss (DE); Wollenschläger, Axel, Dr., D-51373 Leverkusen (DE); Landen, Harald, Dr. Dr., D-51469 Bergisch Gladbach (DE); Schmitt, Franz, D-51465 Bergisch Gladbach (DE)

(56) Entgegenhaltungen:
- EP-A- 0 407 028
- WO-A-92/00771
- WO-A-92/09322
- WO-A-92/18188
- US-A- 2 587 215
- US-A- 5 113 855

## Beschreibung

Die Erfindung betrifft eine Vorrichtung zum Inhalieren eines pulverförmigen, mikronisierten, in eine fließfähige Formulierung gebrachten, pharmakologisch wirksamen Arzneistoffes mit einem Vorratsbehälter für die Arzneistofformulierung, der an seinem unteren Ende mit einer drehbaren oder verschiebbaren, handbetätigten Dosierplatte mit mindestens einer Vertiefung zur wiederholten Aufnahme und Abgabe einer vorgegebenen, reproduzierbaren Arzneistoffmenge aus dem Behälter ganz oder teilweise abgeschlossen ist. Die Arzneiformulierung kann aus reinem mikronisierten Wirkstoff (z. B. weiche Pellets) oder aus einer Mischung mit einem pharmazeutisch akzeptablen Carrier (z. b. Laktose-Monohydrat) bestehen. Außerdem weist die Vorrichtung ein Mundstück zum Inhalieren auf, dem eine Dispergierkammer vorgeschaltet ist.

Bei der Inhalation wird das dosierte Medikament innerhalb des Inhalators in weitgehend respirable Wirkstoffpartikel dispergiert. Es hat sich gezeigt, daß zahlreiche Medikamente vorteilhaft als Aerosol in die Lunge appliziert werden können. Hierdurch ist in vielen Fällen eine besonders schnelle Arzneimittelwirkung bei gleichzeitig sehr geringer und den Patienten wenig belastender Wirkstoffdosis möglich.

Zahlreiche Geräte zur Applikation eines Aerosols wurden bislang entwickelt. So können beispielsweise gelöste Arzneistoffe mittels Druckluft- oder Ultraschallvernebler so fein zerstäubt werden, daß das resultierende Aerosol lungengängig ist. Nachteilig ist der für diese Geräte erhebliche Energiebedarf für die Zerstäubung der Lösungen in respirable Tröpfchen, der große Geräte zur Folge hat und eine Abhängigkeit von externen Energiequellen (z.B. Netzstrom, Akkus) bedeutet. Auch lassen sich viele Arzneistoffe nicht als stabile wäßrige Lösung formulieren.

Ein anderer Weg zur Applikation von Wirkstoffen in die Lunge besteht darin, den Wirkstoff in einem druckverflüssigten Treibmittel zu lösen oder zu dispergieren. Wird diese Lösung oder Dispersion mittels Dosiersystem freigesetzt, so wird der Wirkstoff durch die plötzliche Verdampfung des Treibmittels in feinster Form bereitgestellt und kann inhaliert werden.

Diesen Systemen haften mehrere Nachteile an, z.B.:
- Die erforderliche koordinierte Auslösung des Sprühstoßes mit der Inhalation gelingt sehr vielen Patienten nicht;
- Beitrag zur Umweltbelastung durch Treibgase;
- Kältereiz durch verdunstendes Treibmittel irritiert die Patienten;
- hohe Geschwindigkeit des Aerosols führt zur Abscheidung nennenswerter Mengen im Rachenraum und kann dort Nebenwirkungen begünstigen;
- insgesamt können nur kleine Wirkstoffdosen (um 1 bis 2 mg) verabreicht werden.

Um die Nachteile der Druckgasaerosole zu überwinden, wurden mehrere Pulverinhalatoren entwickelt, bei denen die Inhalationsluft des Patienten zur Dispergierung der Arzneistofformulierung verwendet wird. Hierdurch wird die für den Patienten schwierig zu bewerkstelligende Koordination von Atmung und Auslösung der Dosis entbehrlich.

Ein Beispiel für einen auf dem Markt befindlichen Pulverinhalator ist in den britischen Patentschriften 1 122 284 bzw. 1 331 216 aufgeführt. Dieses Gerät wird mit Hartgelatinekapseln geladen, in denen sich der Wirkstoff in mikronisierter Form befindet. Nach Öffnung der Kapsel durch zwei Nadeln wird durch den Inhalationsatemstrom die Kapsel in Rotation versetzt, wodurch der mikronisierte Wirkstoff in den Luftstrom dispergiert wird und über die Inhalationsluft in die Lungen des Patienten gelangt.

Die bisher bekannten Pulverinhalatoren zeigen noch Nachteile:
- Sie müssen umständlich geladen und gereinigt werden;
- in der Regel sind mehrere Inhalationen erforderlich, um die Kapsel vollständig zu entleeren;
- Patienten können unbeabsichtigt in das Gerät hineinatmen, wodurch sich darin Feuchtigkeit niederschlägt, die einen sehr nachteiligen Effekt auf die Dosierungsgenauigkeit und Dispergierung folgender Wirkstoffdosen hat;
- einige Geräte zeigen einen hohen Atemwiderstand, was von Patienten als zusätzliche Belastung empfunden wird. Andere Geräte erfordern sehr hohe inspiratorische Flußraten, um eine befriedigende Dispergierung zu erzielen, was besonders von kleinen Kindern und älteren Patienten nur ungenügend erreicht wird.

Ein Beispiel für einen Pulverinhalator mit Mehrfachdosierung ist in EP 0 237 507 bzw. in EP 0 069 715 beschrieben. Dort wird der Wirkstoff aus einem Silo über eine perforierte Membran in einen Strömungskanal gebracht und mit der Inhalationsluft des Patienten dispergiert. Jedoch auch dieses Gerät hat noch Nachteile:
- Die Dosierung (Dosierungsgenauigkeit) hängt vom Inhalationsfluß ab. Bei geringen Inhalationsströmen wurden Fehldosierungen und Aussetzer beobachtet, was problematisch ist, da der Patent wegen der geringen applizierten Wirkstoffmengen keine Kontrolle über die Wirkstoffabgabe hat.
- auch die Dispergierung hängt vom Inhalationsfluß ab.
- Patienten können hier, wie auch bei den obengenannten Einfach-Dosiergeräten, unbeabsichtigt in das Gerät hineinatmen, wodurch sich darin Feuchtigkeit niederschlägt, die einen sehr nachteiligen Effekt auf die Dosierung und Dispergierung folgender Wirkstoffdosen hat.

Weiterhin wird in dem US-Patent 2 587 215 ein Inhalator mit Mehrfachdosierung beschrieben, bei dem das zu inhalierende pulverförmige Medikament durch Drehung oder Verschiebung einer Dosierscheibe in eine Misch- bzw. Dispergierkammer gebracht und dort von dem angesaugten Luftstrom aufgewirbelt wird. Das aufgewirbelte Medikament gelangt dann von der Mischkammer direkt in das Mundstück und in die Atemwege des Patienten. Auch bei diesem Gerät besteht das Problem einer reproduzierbaren Dosierung (Dosiergenauigkeit) und Dispergierung.

Die Aufgabe der Erfindung liegt nun darin, einen Pulverinhalator mit Mehrfachdosierung zu entwickeln, der bei geringen Inhalationsströmen bereits zu einer guten Dispergierung der Formulierung führt und bei Erhöhung des Inhalationsstromes ein weitgehend gleichbleibend hohes Dispergierungsniveau beibehält. Unter "Formulierung" wird hier und im folgenden eine Mischung aus Wirkstoff und Hilfsstoff oder auch ein reiner, z. B. agglomerierter, Wirkstoff verstanden. Eine weitere Aufgabe besteht darin, daß bei einem versehentlichen Exhalieren von ungeübten Patienten (Ausatmen in den Inhalator) negative Einflüsse auf die Dosierungsgenauigkeit und die Gerätehygiene vermieden werden.

Diese Aufgabe wird, ausgehend von dem eingangs beschriebenen Pulverinhalator, erfindungsgemäß dadurch gelöst, daß - in Strömungsrichtung gesehen - vor der Dispergierkamer ein mit einem Teil seiner Längsfläche die Dosierplatte partiell überdeckender Beschleunigungskanal angeordnet ist, der tangential in eine zylindrische Verweilzeitkammer mündet, und daß die Verweilzeitkammer über einen zentralen Auslaß mit kleinerem Durchmesser als die zylindrische Verweilzeitkammer mit einer kreisförmigen Austragkammer verbunden ist, an die sich die Dispergierkammer tangential anschließt.

Vorzugsweise ist dem Mundstück ein Diffusor zur Verzögerung des aus der Dispergierkammer austretenden Aerosols vorgeschaltet.

Gemäß einer Weiterentwicklung ist der Beschleunigungskanal durch eine Exhalationssperre in Form einer elastischen Ventilklappe verschließbar, die sich beim Inhalieren öffnet und den Aerosolstrom freigibt.

Vorzugsweise ist diese Ventilklappe als Bandfeder ausgebildet, die gleichzeitig den Beschleunigungskanal und die Vertiefungen in der Dosierplatte abdeckt und verschließt.

Vorteilhaft weist der Vorratsbehälter für den Arzneistoff einen nierenförmigen Querschnitt auf, wobei die Seitenwände senkrecht sind oder mit einer Neigung von maximal 30° zur Senkrechten ausgebildet sind.

Die Dosierplatte ist zweckmäßig so angebracht, daß sie mit einem Teil ihrer Oberfläche den unteren Bereich des Beschleunigungskanals teilweise abschließt, so daß die Vertiefungen in diesem Teilfeld unmittelbar unter dem Beschleunigungskanal liegen, während sich die anderen Vertiefungen unterhalb des Vorratsbehälters befinden.

Gemäß einer bevorzugten Ausführungsform können die Vertiefungen in der Aufsicht in der Dosierplatte eine rechteckige, ovale oder kreisförmige Kontur haben, wobei die größte Breite der Vertiefungen kleiner oder gleich der Breite des Vorratsbehälters ist.

Eine weitere Verbesserung besteht darin, daß sich im Behälter oberhalb der Arzneistoffschüttung ein Trockenmittel in einem separaten Behältnis befindet.

Mit der Erfindung werden folgende Vorteile erzielt: Das Gerät weist für fließfähige Formulierungen (z.B. adhäsive Pulvermischungen) eine sehr hohe Dosiergenauigkeit auf. Überdosierungen durch wiederholtes Drehen des Dosierrades sind hierbei nicht möglich. Schon bei geringen Flußraten wird der Wirkstoff zu einem sehr hohen Anteil in respirable Partikel dispergiert. Bei geeigneten Formulierungen bleibt dieser hohe Anteil auch bei höheren Flußraten nahezu konstant, d.h. die abgegebene Dosis ist in einem relativ weiten Bereich nur wenig vom inspiratorischen Fluß beeinflußt. Durch eine Exhalationssperre wird vermieden, daß Patienten irrtümlich in das Gerät hineinatmen und es hierdurch verunreinigen und gegebenenfalls das Produkt befeuchten. Weiterhin bleibt nach jeder Inhalation nur äußerst wenig Formulierung im Gerät zurück, die leicht durch eine dem Patienten mögliche Reinigung entfernt werden kann. Das Gerät weist bei der Inhalation nur einen mittleren Atemwiderstand auf, der von Patienten nicht als unangenehm empfunden wird.

Im folgenden wird die Erfindung anhand eines in den Zeichnungen dargestellten Ausführungsbeispieles näher erläutert. Es zeigen:
- Fig. 1: eine Seitenansicht (mit Teilschnitt),
- Fig. 2: eine Draufsicht (mit Teilschnitt) und
- Fig. 3: einen vergrößerten Ausschnitt zur Darstellung des Beschleunigungskanals mit der daran anschließenden Verweilzeitkammer (Schnitt A-A' in Fig. 2).

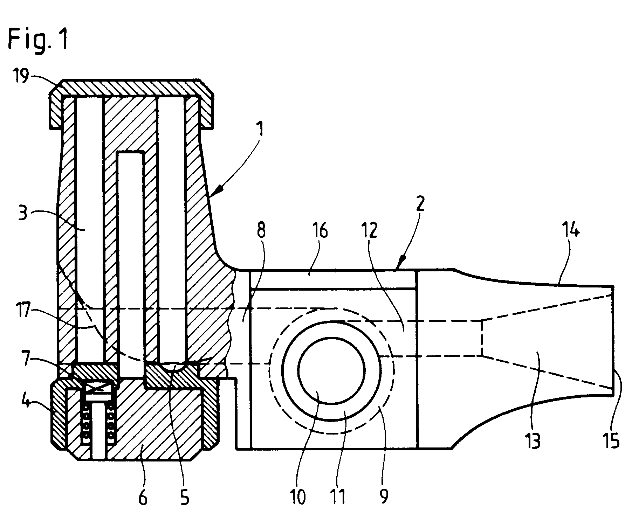

Der Pulverinhalator gemäß Fig. 1 besteht grundsätzlich aus einem in Gebrauchsstellung vertikal stehenden Dosierteil 1 und einem daran anschließenden horizontalen Dispergierteil 2. Im Dosierteil 1 ist ein halbkreisförmiger bzw. nierenförmiger Vorratsbehälter 3 für die fließfähige Arzneistofformulierung untergebracht. Am unteren Ende des Dosierteils 1 ist ein um die Längsachse drehbares Dosierrad 4 mit Vertiefungen 5 angeordnet. Die Vertiefungen 5 sind in die horizontale Oberfläche des Dosierrades 4 eingearbeitet. Das Dosierrad 4 kann sich um einen zylindrischen, am unteren Ende des Siloteils angebrachten Lagerblock 6 drehen. Zur Fixierung des Dosierrades 4 in bestimmten vorgegebenen Winkelpositionen ist innerhalb des Lagerblocks 6 eine Rastsperre 7 vorgesehen. Die äußere Mantelfläche des Dosierrades 4 ist mit einer Riffelung versehen, um die manuelle Betätigung zu erleichtern.

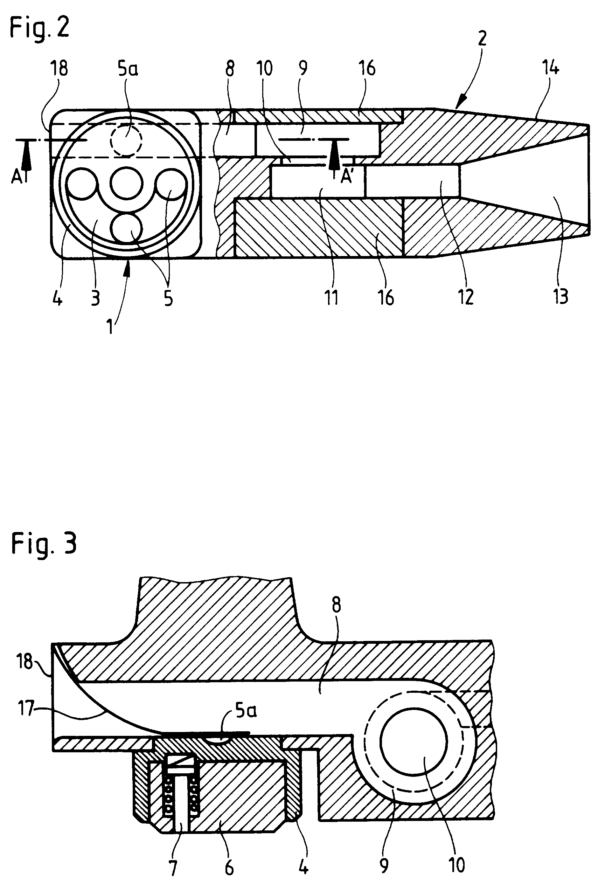

Oberhalb des Dosierrades 4 (siehe auch Fig. 2 und 3) erstreckt sich längs durch das Dispergierteil 2 ein seitlich zur Mittelachse versetzter Beschleunigungskanal 8. Aus Fig. 2 ist in Verbindung mit Fig. 1 ersichtlich, daß sich der Beschleunigungskanal 8 über einen Teil der Dosierradoberfläche hinweg erstreckt. Gemäß Fig. 2 weist das Dosierrad 4 vier Vertiefungen 5 auf, wobei eine der Vertiefungen 5a gerade unterhalb des Beschleunigungskanals 8 liegt, während sich die anderen drei Vertiefungen unterhalb des Vorratsbehälters 3 befinden. Anstelle mehrerer, gleichmäßig über den Umfang verteilter Vertiefungen kann das Dosierrad 4 auch nur eine einzige Vertiefung 5 aufweisen. Der Beschleunigungskanal 8 mündet tangential in eine zylindrische Verweilzeitkammer 9, die über einen zentralen Auslaß 10 mit kleinerem Durchmesser als die zylindrische Verweilkammer 9 mit einer kreisförmigen Austragskammer 11 verbunden ist. An die Austragskammer 11 schließt sich ebenfalls tangential eine Dispergierkammer 12 an, die in einen im Mundstücksbereich liegenden, konisch erweiterten Diffusor 13 übergeht. Durch den Diffusor 13 im Mundstück 14 wird das aus der Dispergierkammer 12 einströmende Aerosol in Richtung zur Austrittsöffnung 15 verzögert. Die Seitenwände des Dispergierteils 2 sind in Form einer abnehmbaren Klammer 16 ausgebildet, so daß (nach dem Abziehen der Klammer 16) die Kammern 9, 11, 12, 13 für Reinigungszwecke zugänglich sind.

Fig. 3 zeigt noch einmal das Dosierrad 4 mit dem Lagerblock 6 und dem oberhalb des Dosierrades angeordneten, seitlich versetzten Beschleunigungskanal 8. Im Beschleunigungskanal 8 ist eine Exhalationssperre oder Ventilklappe in Form einer den gesamten Querschnitt des Beschleunigungskanals 8 ausfüllenden Bandfeder 17 angebracht, die im Ruhezustand diagonal im Kanal 8 liegt. Die Bandfeder 17 kann auch durch ein federbelastetes, starres Blech mit einer fest vorgegebenen Form ersetzt werden. Sie ist so im Beschleunigungskanal angeordnet, daß sie im Ruhezustand nicht nur den Kanal zur Ansaugöffnung 18 hin abschließt, sondern mit ihrem Ende auch die im Bereich des Beschleunigungskanals 8 liegende Vertiefung 5a des Dosierrads 4 abdeckt. Dadurch wird erreicht, daß in den Vertiefungen 5 befindliches Medikament auch bei einer Drehung des Inhalators nicht herausfallen kann. Gleichzeitig verhindert die diagonal stehende Bandfeder 17, daß durch das Gerät exhaliert werden kann, da der Beschleunigungskanal 8 durch die Bandfeder 17 dicht versperrt wird. Auf diese Weise kann auch bei zufälligen und unbeabsichtigten Exhalationen (Ausatmungen) keine Feuchtigkeit in das dosierbereite Medikament in die Vertiefungen 5 gelangen. Beim Inhalieren wird dagegen die Bandfeder 17 durch den beim Inhalationsvorgang entstehenden Unterdruck angehoben, so daß der Beschleunigungskanal 8, abhängig vom inhalierten Volumenstrom, und gleichzeitig auch die Vertiefung 5a freigegeben werden, so daß die Vertiefung 5a mit hoher Geschwindigkeit überströmt wird. Aufgrund der Turbulenz der Strömung wird das pulverförmige Medikament aus der Vertiefung 5a in den Beschleunigungskanal 8 ausgetragen.

Nachfolgend werden der Inhalationsvorgang und die Wirkungsweise des Pulverinhalators beschrieben:
Gemäß Fig. 1 befindet sich der zu inhalierende pulverförmige, mikronisierte, formulierte Arzneistoff im Vorratsbehälter 3. Ein Teil der Vertiefungen 5 im Dosierrad 4 (gemäß Fig. 2 alle, bis auf die Vertiefung 5a) steht mit dem Behälter 3 in Verbindung und wird aufgefüllt, wenn sich der Dosierteil 1 in Vertikallage befindet. Vor einer Inhalation dreht der Patient das Dosierrad 4 mit der Hand bis zur nächsten Einrastung weiter. Dabei wandert eine mit dem pulverförmigen Arzneistoff gefüllte Vertiefung 5 aus dem Bereich unterhalb des Vorratsbehälters 3 in den Bereich des Beschleunigungskanals, wobei sich das Dosierrad 4 mit dieser Vertiefung 5a in Fortsetzung der unteren Längsfläche des Beschleunigungskanals 8 erstreckt. Bei der Ausführung mit einer einzigen Vertiefung müßte das Dosierrad in diesem Fall um einen größeren Winkel weitergedreht werden, bis diese Vertiefung mit dem Beschleunigungskanal 8 fluchtet.

Bei der Inhalation wird, wie oben beschrieben, die Bandfeder 17 angehoben und das Medikament aus der Vertiefung 5a des Dosierrades 4 in den Beschleunigungskanal 8 transportiert. Von dort gelangt das pulverförmige Medikament tangential in die Verweilzeitkammer 9. Durch die verzögerte Freisetzung aus der Verweilzeitkammer 9 unterliegt die Wirkstofformulierung längere Zeit den dispergierenden Kräften, so daß eine insgesamt bessere Dispergierung in respirable Wirkstoffpartikel erreicht wird. Die bereits dispergierten Partikel gelangen über den zentralen Auslaß 10 in die Austragskammer 11.

Durch die Verweilzeitkammer 9 wird die Wirkstoffabgabe also über einen gewissen Zeitraum verzögert. Hierdurch wird, im Unterschied zu Pulverinhalatoren nach dem Stand der Technik, nicht bereits ein Großteil des Medikaments zu Beginn der Inhalation bei relativ geringen Inhalationsgeschwindigkeiten und damit in einem schlechten Dispergierzustand abgegeben.

Aus der Austragskammer 11 wird das Medikament durch die tangential angesetzte Dispergierkammer 12 beschleunigt und damit zusätzlich dispergiert. Im anschließenden, sich konisch erweiternden Diffusor 13 im Mundstück 14 wird die Partikelgeschwindigkeit bis zum Austritt (Austrittsöffnung 15) verzögert, was die Wahrscheinlichkeit einer Prallabscheidung relativ kleiner Partikel im Rachenraum verringert.

Der Vorratsbehälter 3 im Dispergierteil 1 wird mit einem Silodeckel 19 verschlossen. Im Deckel 19 kann ein kleines Behältnis mit einem Trockenmittel untergebracht werden, um die Arzneistofformulierung im Vorratsbehälter 3 vor Feuchtigkeit zu schützen.

## Patentansprüche

1. Vorrichtung zum Inhalieren eines pulverförmigen, mikronisierten und formulierten, pharmakologisch wirksamen Arzneistoffes in Form eines Aerosols, bestehend aus einem Vorratsbehälter (3) für den Arzneistoff, der an seinem unteren Ende mit einer drehbaren oder verschiebbaren, handbetätigten Dosierplatte (4) mit mindestens einer Vertiefung (5) zur wiederholten Aufnahme und Abgabe einer vorgegebenen, reproduzierbaren Arsneistoffmenge aus dem Behälter (3) ganz oder teilweise abgeschlossen ist, und einem Mundstück (14) zum Inhalieren, dem eine Dispergierkammer (12) vorgeschaltet ist, dadurch gekennzeichnet, daß - in Strömungsrichtung gesehen - vor der Dispergierkammer (12) ein mit einem Teil seiner Längsfläche die Dosierplatte (4) partiell überdeckender Beschleunigungskanal (8) angeordnet ist, der tangential in eine zylindrische Verweilzeitkammer (9) mündet, und daß die Verweilzeitkammer (9) über einen zentralen Auslaß (10) mit kleinerem Durchmesser als die zylindrische Verweilzeitkammer (9) mit einer kreisförmigen Austragskammer (11) verbunden ist, an die sich die Dispergierkammer (12) tangential anschließt.

2. Vorrichtung nach Anspruch 1, dadurch gekennzeichnet, daß der Austrittsöffnung (15) im Mundstück (14) ein Diffusor (13) zur Verzögerung des aus der Dispergierkammer (12) austretenden Aerosols vorgeschaltet ist.

3. Vorrichtung nach Anspruch 1 bis 2, dadurch gekennzeichnet, daß der Beschleunigungskanal (8) durch eine Exhalationssperre in Form einer elastischen Ventilklappe (17) verschließbar ist, die sich beim Inhalieren öffnet und den Aerosolstrom freigibt.

4. Vorrichtung nach Anspruch 3, dadurch gekennzeichnet, daß die Ventilklappe als Bandfeder (17) ausgebildet ist, die gleichzeitig den Beschleunigungskanal (8) und die Vertiefungen (5) in der Dosierplatte (4) abdeckt und verschließt.

5. Vorrichtung nach Anspruch 1 bis 4, dadurch gekennzeichnet, daß der Behälter (3) einen nierenförmigen Querschnitt aufweist, wobei die Seitenwände senkrecht oder mit einer Neigung von maximal 30° zur Senkrechten ausgebildet sind.

6. Vorrichtung nach Anspruch 1 bis 5, dadurch gekennzeichnet, daß ein Teilfeld der Dosierplatte (4) den unteren Bereich des Beschleunigungskanals (8) teilweise abschließt.

7. Vorrichtung nach Anspruch 1 bis 6, dadurch gekennzeichnet, daß die Vertiefungen (5) in der Dosierplatte (4) eine rechteckige, ovale oder kreisförmige Kontur haben und ihre größte Breite kleiner oder gleich der Breite des Vorratsbehälters (3) ist.

8. Vorrichtung nach Anspruch 1 bis 7, dadurch gekennzeichnet, daß sich im Behälter (3) oberhalb der Arzneistoff-Schüttung ein Trockenmittel in einem separaten Behältnis im Silodeckel (19) befindet.

## Claims

1. Device for inhaling a powdery, micronized and formulated, pharmacologically active drug in the form of an aerosol, comprising of a supply container (3) for the drug, which supply container is completely or partially closed off at its lower end by a rotatable or displaceable, manually actuated metering plate (4) with at least one depression (5) for the repeated reception and delivery of a predetermined, reproducible quantity of the drug from the container (3), and a mouthpiece (14) for inhaling, upstream of which a dispersing chamber (12) is connected, characterized in that - seen in the flow direction - an acceleration channel is arranged ahead of the dispersing chamber (12), which acceleration channel (8) partially covers the metering plate (4) with part of its longitudinal surface and opens out tangentially into a cylindrical dwell-time chamber (9) and in that the dwell-time chamber (9) is connected via a central outlet (10) of smaller diameter than the cylindrical dwell-time chamber (9) to a circular discharge chamber (11) which the dispersing chamber (12) adjoins tangentially.

2. Device according to Claim 1, characterized in that a diffuser (13) is connected upstream of the outlet aperture (15) in the mouthpiece (14) to delay the aerosol emerging from the dispersing chamber (12).

3. Device according to Claims 1 to 2, characterized in that the acceleration channel (8) can be closed by an exhalation barrier in the form of an elastic valve flap (17) which opens during inhalation and releases the aerosol flow.

4. Device according to Claim 3, characterized in that the valve flap is constructed as a volute spring (17) which simultaneously covers and closes the acceleration channel (8) and the depressions (5) in the metering plate (4).

5. Device according to Claims 1 to 4, characterized in that the container (3) has a kidney-shaped cross-section, the side walls being vertical or being constructed at a maximum inclination of 30° to the vertical plane.

6. Device according to Claims 1 to 5, characterized in that a partial area of the metering plate (4) partially closes off the lower region of the acceleration channel (8).

7. Device according to Claims 1 to 6, characterized in that the depressions (5) in the metering plate (4) have a rectangular, oval or circular contour, the greatest width of the depressions being smaller than or equal to the width of the supply container (3).

8. Device according to Claims 1 to 7, characterized in that there is a desiccant in a separate receptacle in the reservoir lid (19) in the container (3) above the bulk of the drug.

## Revendications

1. Dispositif pour l'inhalation sous forme d'un aérosol d'un médicament pharmacologiquement efficace, pulvérulent, micronisé et formulé, constitué par un récipient de réserve (3) pour le médicament, qui, à son extrémité inférieure, est fermé, en tout ou en partie, par une plaque de dosage (4) rotative ou mobile, qui peut être manipulée à la main, comportant au moins un renfoncement (5) pour le prélèvement et la libération répétés d'une quantité de médicament prédéfinie, reproductible, hors du récipient (3), et par un embout (14) pour l'inhalation en amont duquel est montée une chambre (12) de mise en dispersion, caractérisé en ce que - vu dans la direction d'écoulement - est disposé avant la chambre (12) de mise en dispersion, un canal d'accélération (8) recouvrant partiellement la plaque de dosage (4) avec une partie de sa surface longitudinale, qui aboutit en direction tangentielle dans une chambre cylindrique (9) de temps de séjour et en ce que la chambre de temps de séjour (9) est reliée, via une sortie centrale (10) dont le diamètre est inférieur à celui de la chambre cylindrique (9) de temps de séjour, à une chambre d'évacuation circulaire (11) à laquelle se raccorde, de manière tangentielle, la chambre (12) de mise en dispersion.

2. Dispositif selon la revendication 1, caractérisé en ce que, en amont de l'ouverture d'évacuation (15), est monté dans l'embout (14) un diffuseur (13) pour ralentir l'aérosol qui quitte la chambre (12) de mise en dispersion.

3. Dispositif selon les revendications 1 à 2, caractérisé en ce que le canal d'accélération (8) peut être fermé par un blocage de l'exhalation sous forme d'un clapet (17) qui s'ouvre lors de l'inhalation et qui libère le courant d'aérosol.

4. Dispositif selon la revendication 3, caractérisé en ce que le clapet est réalisé sous la forme d'un ressort à ruban (17) qui recouvre et ferme simultanément le canal d'accélération (8) et les renfoncements (5) dans la plaque de dosage (4).

5. Dispositif selon les revendications 1 à 4, caractérisé en ce que le récipient (3) présente une section transversale réniforme, les parois latérales étant réalisées à la verticale ou avec une inclinaison maximale de 30° par rapport à la verticale.

6. Dispositif selon les revendications 1 à 5, caractérisé en ce qu'un champ partiel de la plaque de dosage (4) ferme partiellement la zone inférieure du canal d'accélération (8).

7. Dispositif selon les revendications 1 à 6, caractérisé en ce que les renfoncements (5) dans la plaque de dosage (4) possèdent un contour rectangulaire, ovale ou circulaire, leur largeur maximale étant inférieure ou égale à la largeur du récipient de réserve (3).

8. Dispositif selon les revendications 1 à 7, caractérisé en ce que, dans le récipient (3), au-dessus de la distribution du médicament, se trouve un desséchant dans un récipient séparé placé dans le couvercle de silo (19).
